# EUROPEAN PATENT SPECIFICATION

(11) **EP 0 461 705 B1**
(45) Date of publication and mention of the grant of the patent: **15.03.2000**
(21) Application number: 91201383.6
(22) Date of filing: 05.06.1991
(51) Int. Cl.: A61K 31/505

(54) **Agents for treating addiction to habit-forming drugs**
Wirkstoff zur Behandlung der Drogenabhängigkeit
Agents actifs pour le traitement de l'asservissement aux drogues

(30) Priority: 13.06.1990 US 537175
(43) Date of publication of application: 18.12.1991
(73) Proprietor: JANSSEN PHARMACEUTICA N.V., 2340 Beerse (BE)
(72) Inventor: Meert, Theo Frans, B-2840 Rumst (BE)
(74) Representative: Wante, Dirk

(56) References cited:
- PSYCHOPHARMACOLOGY, vol. 100, no. 2, February 1990, pages 258-266, Springer-Verlag; B.S. NEAL et al.: "The serotonin2 antagonist ritanserin blocks quasi-morphine withdrawal at a time when mianserin is no longer effective"
- IDEM
- ARCH. INT. PHARMACODYN THER., vol. 290. no. 1, 1987, pages 154-155; J. DUYSENS et al.: "Pharmacological suppression of the hindlimb withdrawal reflex of the rat; a comparison between serotonergic antagonists and an adrenergic agonist"
- IDEM
- BRIT. J. PHARMACOL., vol. 89, proc. suppl., 1986, page 647P; S.L. HANDLEY et al.: "Ritanserin reduces morphine, and clonidine, withdrawal tics"
- IDEM
- PHARMACOPSYCHIATRY, vol. 24, no. 5, September 1991, pages 159-163, Georg Thieme Verlag, Stuttgart, DE; T.F. MEERT et al.: "Ritanserin reduces abuse of alcohol, cocaine, and fentanyl in rats"
- EUR. J. PHARMACOLOGY, vol. 183, no. 5, July 1990, page 1924; T.F. MEERT et al.: "The preference for alcohol and cocaine is virtually abolished by the 5-HT2 receptor antagonist ritanserin"

## Description

It is generally known that the repeated consumption of habit-forming drugs such as alcohol, minor tranquilizers, stimulantia, opiates, hallucinogens, nicotine in many instances leads to different degrees of addiction. Typically, such addiction is characterized by a need or desire to continue the use of the drug and to obtain it, not seldom by all means, and further by a tendency to increase its dosage. This usually results in a psychological and usually a physiological dependence on the effects of such drugs and eventually has a detrimental effect on the addicted individual and on society.

Once a certain degree of addiction is reached, abstention of habit-forming drugs becomes a serious problem and is often accompanied by undesirable physical and/or psychic symptoms. Therefore, an agent decreasing or overcoming such addiction and, if possible, alleviating or removing the symptoms related to the withdrawal of such habit-forming drugs would be highly welcomed, not only by drug addicts, but also by society in general.

In Psychopharmacology, Vol. 100, February 1990, p. 258-266, Neal et al. disclose the use of ritanserin, administered prior to injection of a drug (isobutylmethylxanthine-"IBMX") that induces quasi-morphine withdrawal syndrome ("QMWS"), to block the QMWS. The test animals are not addicted to any habit-forming drugs (the test animals were "opiate naive" rats). The symptoms that were treated were those that are characteristic of the acute or detoxification phase of drug withdrawal. There is no teaching or suggestion of the use of ritanserin to alleviate the characteristic symptoms that occur during the chronic phase.

In Arch. Int. Pharmacodyn. Ther. 290(1), 1987 p. 154-155 (D2), ritanserin is used in the "nociceptive flexor reflex"-test in rats, which is a model to investigate the alleviation of pain and has no bearing on the treatment of addiction to habit-forming drugs.

Brit. J. Pharmacology, 89, 1986, p. 647 (D3) discloses the use of ritanserin to reduce tics that occur upon withdrawal from morphine. The article also describes experiments with clonidine, which is not an addictive drug. In the experiments described in D3, morphine is administered to induce physical addiction, naloxone is given to induce a condition called "precipitated morphine abstinence", and ritanserin or vehicle are administered to observe the presence or absence of an effect on the induced withdrawal symptoms. The head twitches or tics were counted 22 hours after withdrawal. The tics measured in the experiments described in D3 are physical symptoms that occur during the acute or detoxification stage of drug withdrawal. Thus, this article discloses the use of ritanserin to treat acute phase withdrawal symptoms, but does not teach or suggest that ritanserin can be used to treat chronic phase withdrawal symptoms such as the craving for habit forming drugs.

It now has been found that certain 4-[bis(halophenyl)methylene]-1-piperidinyl derivatives alleviate, suppress or overcome addiction to habit-forming drugs.

The present invention therefore is concerned with the use of a compound of formula 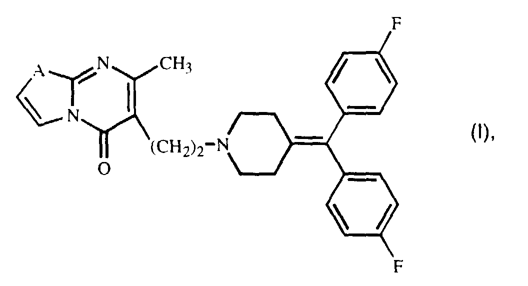 or a pharmaceutically acceptable acid addition salts thereof, wherein A represents -CH=CH- or -S-, for the manufacture of a medicament for alleviating, suppressing or overcoming addiction to habit forming drugs.

The compound of formula (I) wherein A is -S-, namely 6-[2-[4-[bis(4-fluorophenyl)-methylene]-1-piperidinyl]ethyl]-7-methyl-5H-thiazolo[3,2-a]pyrimidin-5-one is generically designated as ritanserin. The compound of formula (I) wherein A is -CH=CH-, namely 3-[2-[4-[bis(4-fluorophenylmethylene]-1-piperidinyl]ethyl]-2-methyl-4H-pyrido[1,2-a]pyrimidin-4-one is generically designated as seganserin. These compounds as well as their preparation and pharmacological properties are known from US-4,485,107.

The aforementioned term "pharmaceutically acceptable acid addition salt" is meant to comprise those salts obtained by treating the base form of the active ingredients of formula (I) with appropriate acids, such as, for example, inorganic acids, e.g. hydrochloric, hydrobromic and the like acids, sulfuric acid, nitric acid, phosphoric acid and the like; or organic acids, such as, for example, acetic, propanoic, hydroxyacetic, 2-hydroxypropanoic, 2-oxopropanoic, ethanedioic, propanedioic, butanedioic, (Z)-2-butenedioic, (E)-2-butenedioic, 2-hydroxybutanedioic, 2,3-dihydroxybutanedioic, 2-hydroxy-1,2,3-propanetricarboxylic, methanesulfonic, ethanesulfonic, benzene-sulfonic, 4-methylbenzenesulfonic, cyclohexanesulfamic, 2-hydroxybenzoic, 4-amino-2-hydroxybenzoic and the like acids. The term "pharmaceutically acceptable acid addition salt" also comprises the solvates which the compounds of formula (I) may form and said solvates are intended to be included within the scope of the present invention. Examples of such solvates are e.g. the hydrates, alcoholates and the like.

Habit-forming drugs as referred hereinabove comprise various agents such as alcohol; cocaine, or fentanyl. Certain of the aforementioned habit-forming drugs are also referred to as drugs of abuse (or simply as "drugs") or as narcotics.

An individual continuously using these habit-forming drugs usually becomes addicted thereto. In some cases, addiction arises even after having taken the drug only a few times. Individuals being addicted to habit-forming drugs are confronted with a need, desire or longing for such drugs, the strength thereof depending on the individual, the degree of addiction or the kind of drug. In some instances, this need, desire or longing is accompanied by (or causes) a neglecting of the addicted individual's well-being and health, e.g. a loss of appetite, a reduction of mental awareness, social disbehavior and the like. The latter factors too are to be considered as undesired side effects of drug addiction which can be treated by the agents of the present invention.

Individuals thus having become addicted and wanting, or being necessitated, to discontinue the use of habit-forming drugs usually find themselves confronted with a number of undesired symptoms, in particular a continuous desire or longing for the habit-forming drug having caused the addiction.

The compounds of formula (I) and their acid addition salts are preferably administered formulated in usual pharmaceutical compositions comprising an effective amount of the active ingredient and a suitable pharmaceutically acceptable carrier. Depending on the mode of administration, such compositions may take a variety of forms, e.g. tablets, solutions for parenteral administration, capsules, solutions or suspensions for oral intake, powders and the like. Such compositions are prepared by intimately mixing the active ingredient with one or more suitable carriers and converting this mixture into a form suitable for administration.

The dose to be administered may vary upon the individual which is treated, and is dependent on his or her body size, the degree of addiction and in particular on the kind of habit-forming drug causing the addiction.

A suitable daily dose is contemplated to vary between about 0.1 mg/kg and 50 mg/kg body weight, and in particular between 0.5 and 10 mg/kg body weight, more in particular between 0.5 and 5 mg/kg body weight.

The effectiveness of the compounds of formula (I), as defined above, in overcoming addiction to habit-forming drugs can be demonstrated in the following test procedures.

### Example 1

Rats forced to drink 3% alcohol for a week followed by a week of alcohol withdrawal, reveal a high preference for alcohol when given the choice between 3% alcohol and water.

The compound ritanserin, given during both the period of alcohol withdrawal and choice between alcohol and water, results in a dose-related reduction in the preference for alcohol. Doses ³ 0.63 mg/kg body weight ritanserin given subcutaneoulsy once a day, reduced total alcohol consumption as well as the relative preference for alcohol. At 10 mg/kg ritanserin, the highest dose tested, alcohol consumption was reduced with 58% and none of the tested animals had a pronounced preference for alcohol anymore. Simultaneous with the reduction in alcohol consumption, there was an increased water intake, keeping total fluid intake constant. Ritanserin's activity was observed from the first day of choice and the drug remained active during all five test days.

These results indicate that ritanserin can reduce alcohol intake and the alcohol preference without interfering with total fluid intake and without creating an alcohol aversion.

### Example 2

Rats drinking 0.1 mg/ml cocaine for a week, followed by a week of cocaine withdrawal, reveal a preference for cocaine when given the choice between 0.1 mg/ml cocaine and water in the third week. Ritanserin, given once daily during both the period of cocaine withdrawal and choice between cocaine and water, reduces the preference for cocaine as well as the total amount of cocaine consumed. At 10 mg/kg ritanserin, cocaine intake was reduced by about 30%. Reduction in cocaine intake was accompanied by an increased water consumption keeping total fluid intake constant. Reduction in preference for cocaine remained present at doses down to 2.5 mg/kg ritanserin. However, at the tested doses a complete cocaine aversion was not observed, because the test animals continued to consume some quantity of cocaine. Ritanserin's activity was present from the first day of choice and the drug remained active during the entire period of choice.

### Example 3

Rats given the choice between fentanyl and water developed a preference for fentanyl after a first period of exposure to fentanyl alone. Ritanserin was administered subcutaneously once daily. At 2.5 mg/kg ritanserin, the highest dose tested, fentanyl intake and fentanyl preference reduced by 50 and 33%, respectively. A reduction of fentanyl preference was observed at doses down to 0.04 mg/kg and was present from the first day of treatment. The reduction in fentanyl intake was compensated by an increase in water drinking. At no time there was a systematic interference of ritanserin with consumatory physiological processes nor did ritanserin create any fentanyl aversion. Furthermore, ritanserin did not affect the discriminative stimulus properties of fentanyl.

## Claims

1. Use of a compound of formula 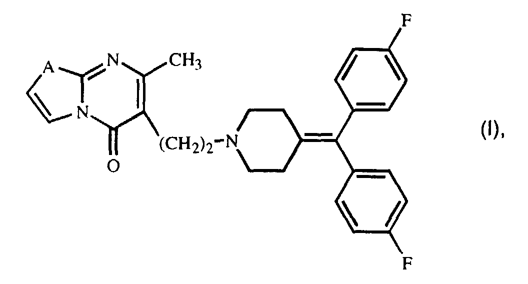 or a pharmaceutically acceptable acid addition salt thereof, wherein A represents -CH=CH- or -S-, for the manufacture of a medicament for alleviating, suppressing or overcoming addiction to habit-forming drugs.

2. Use of a compound of formula (I) wherein A is S.

3. Use according to claim 2 wherein the habit-forming drug is alcohol, cocaine or fentanyl.

## Patentansprüche

1. Verwendung einer Verbindung der Formel 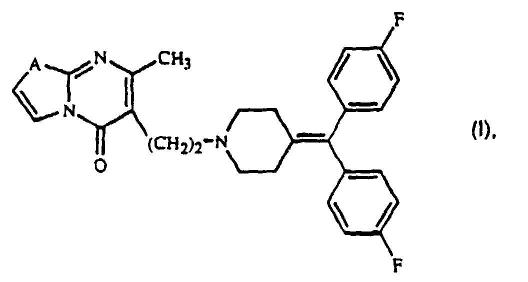 oder eines pharmazeutisch unbedenklichen Säureadditionssalzes davon, wobei A für -CH=CH- oder -S- steht, zur Herstellung eines Arzneimittels zur Abschwächung, Unterdrückung oder Überwindung der Abhängigkeit von gewohnheitsbildenden Arzneistoffen.

2. Verwendung einer Verbindung der Formel (I), worin A für S steht.

3. Verwendung nach Anspruch 2, wobei es sich bei dem gewohnheitsbildenden Arzneistoff um Alkohol, Kokain oder Fentanyl handelt.

## Revendications

1. Utilisation d'un composé de formule 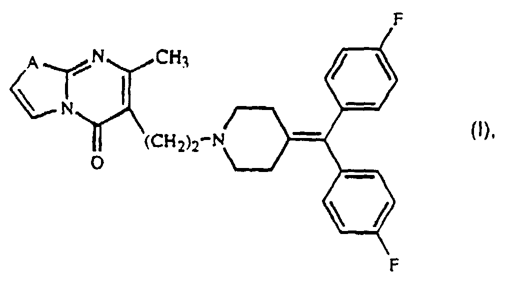 ou d'un sel d'addition pharmaceutiquement acceptable de celui-ci à un acide, dans laquelle A représente -CH=CH- ou -S-, pour l'élaboration d'un médicament destiné à atténuer, supprimer ou remédier à une dépendance vis-à-vis de drogues créant une accoutumance.

2. Utilisation d'un composé de formule (I), dans laquelle A est S.

3. Utilisation selon la revendication 2, dans laquelle la drogue créant une accoutumance est l'alcool, la cocaïne, ou le fentanyl.
